# EUROPEAN PATENT SPECIFICATION

(11) **EP 2 517 728 B1**
(45) Date of publication and mention of the grant of the patent: **28.01.2015**
(21) Application number: 12175765.2
(22) Date of filing: 18.02.2004
(51) Int. Cl.: A61K 45/06, A61K 31/4178, A61P 27/02, A61P 43/00

(54) **Olmesartan medoxomil for preventing or treating angiogenic eye diseases**
Olmesartan medoxomil zur Vorbeugung oder Behandlung angiogener Augenerkrankungen
Olmesartane medoxomil pour la prévention ou le traitement des maladies oculaires angiogènes

(30) Priority: 15.04.2003 JP 2003109918
(43) Date of publication of application: 31.10.2012
(62) Divisional of application: 04712221.3
(73) Proprietor: Daiichi Sankyo Company, Limited, Tokyo 103-8426 (JP)
(72) Inventor: Yokoyama, Tomihisa, Tokyo, 140-8710 (JP); Inoue, Tatsuya, Tokyo, 140-8710 (JP)
(74) Representative: Fairbairn, Angus Chisholm

(56) References cited:
- EP-A1- 0 503 785
- Michel Lonchampt ET AL: "Hyperoxia/normoxia-driven retinal angiogenesis in mice: a role for angiotensin II.", Investigative ophthalmology & visual science United States Feb 2001, 1 January 2001 (2001-01-01), pages 429-432, XP55038458, Retrieved from the Internet: URL:http://www.iovs.org/content/42/2/429.f ull.pdf [retrieved on 2012-09-18]
- YANAGISAWA H ET AL: "Nonpeptide Angiotensin II Antagonists. Synthesis, Biological Activities, and Structure-Activity Relationships of Imidazole-5-Carboxylic Acids Bearing Alkyl, Alkenyl and Hydroxyalkyl Substitutents at the 4-Position and Their Related Compounds", JOURNAL OF MEDICINAL CHEMISTRY, AMERICAN CHEMICAL SOCIETY, US, vol. 39, no. 1, 5 January 1996 (1996-01-05), pages 323-338, XP002144317, ISSN: 0022-2623, DOI: 10.1021/JM950450F

## Description

### [Technical field]

The present invention relates to a medicament for the prevention or treatment of intraocular angiogenic diseases comprising an angiotensin II receptor antagonist as its active ingredient.

### [Background art]

Angiotensin II receptor antagonists are used as therapeutic agents of circulatory diseases including heart diseases such as cardiac hypertrophy, cardiac failure or myocardial infarction, cerebral apoplexy or nephritis, as well as hypertension, and it is thought that their mechanism of action is the result of inhibiting binding of angiotensin II, which has potent vasoconstrictive action, to angiotensin II receptors.

The actions of angiotensin II receptor antagonists on intraocular angiogenic diseases are known to involve angiotensin II receptor antagonists improving changes in electroretinograms in an animal model of diabetes (see, for example, Diabetologia, 44, 883-888, 2001), and suppressing neovascularization of the retina in an animal model of proliferative retinopathy (see, for example, Investigative Ophthalmology & Visual Science, 42, 429-432, 2001). In addition, compounds having angiotensin II antagonistic action are known to be effective in the prevention or treatment of simple retinopathy and pre-proliferative retinopathy, which are early and intermediate disease states of diabetic retinopathy (see, for example, Japanese Patent Application (Kokai) No. 2001-10975). However, it has not conventionally been known that compounds having angiotensin II antagonistic action are effective in the prevention or treatment of intraocular angiogenic diseases such as proliferative retinopathy, which occurs in the late stage of diabetic retinopathy or retinal vein occlusion. Furthermore, vascular endothelial growth factor (VEGF) is considered to play an important role as a regulatory factor of neovascularization in intraocular angiogenic diseases (Molecular Medicine, 35, 1252-1260, 1998; Journal of Cellular Physiology, 184, 301-310, 2000).

### [Disclosure of the invention]

An object of the present invention is to provide a useful medicament for the prevention or treatment of retinal ischemia. As a result of extensive research to achieve the object described above, the inventors of the present invention have found that an angiotensin II receptor antagonist is highly effective in the prevention or treatment of intraocular angiogenic diseases such as proliferative retinopathy or retinal vein occlusion. The present invention was completed based on these findings described above.

Specifically, the present invention provides a medicament comprising (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate, as its active ingredient, for use in the prevention or treatment of an intraocular angiogenic disease that is proliferative retinopathy, retinal vein occlusion, retinal artery occlusion or age-related macular degeneration, by oral administration.

In another aspect, the use of (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate, in the manufacture of a medicament for the prevention or treatment of an intraocular angiogenic disease that is proliferative retinopathy, retinal vein occlusion, retinal artery occlusion or age-related macular degeneration, by oral administration is provided by the present invention.

### [Best mode for carrying out the invention]

In the present invention, an angiotensin II receptor antagonist refers to a substance capable of competitively or non-competitively inhibiting the binding of angiotensin II to an angiotensin II receptor. Whether or not a certain substance has angiotensin II receptor antagonistic action can easily be confirmed by a person with ordinary skill in the art in accordance with, for example, the method described in the specification of US Patent No. 5,616,599.

The angiotensin II receptor antagonist used in the medicament of the present invention is a pharmacologically acceptable ester, specifically the (5-methyl-2-oxo-1,3-dioxolen-4-yl)methyl ester, of 4-(1-hydroxy-1-methylethyl)-2-propyl-1-[2'-(1H-tetrazol-5-yl)biphenyl-4-ylmethyl]imidazole-5-carboxylic acid. 4-(1-Hydroxy-1-methylethyl)-2-propyl-1-[2'-(1H-tetrazol-5-yl)biphenyl-4-ylmethyl]imidazole-5-carboxylic acid is known, and can easily be obtained by the method described in, for example, Japanese Patent Application (Kokai) No. Hei 5-78328 (US Patent No. 5,616,599).

The medicament of the present invention can be used for the prevention or treatment of intraocular angiogenic diseases. In the present specification, the phrase "prevention or treatment" includes the amelioration or cure of diseases, as well as suppression of the progress or inhibition of the onset of diseases, and the prevention of its recurrence. The phrase "prevention or treatment" should not be interpreted in a limiting manner, but rather should be interpreted in the broadest sense for all meanings for which it is used.

Intraocular angiogenic diseases are the diseases that exhibit pathology primarily consisting of neovascularization within the eye, which include, for example, proliferative retinopathy, retinal vein occlusion, retinal artery occlusion or age-related macular degeneration. Proliferative retinopathy is a form of diabetic retinopathy, and is defined as being a disease state that follows an early disease stage in the form of simple retinopathy, and an intermediate disease stage in the form of pre-proliferative retinopathy (Diabetes, 42, 409-410 (1999)). It can be distinctly classified by experienced physicians. Furthermore, the term "proliferative retinopathy" includes the entire series of disease stages that lead to retinal detachment due to neovascularization within the retina in general.

The medicament of the present invention is to be administered orally. As suitable dosage forms for oral administration, powders, granules, tablets and capsules can be listed, but are not restricted to these forms. One or two or more types of preparation additives can be used when preparing the dosage forms.

As preparation additives, for example, excipients (for example, organic excipients including sugar derivatives such as lactose, sucrose, glucose, mannitol or sorbitol; starch derivatives such as corn starch, potato starch, α-starch or dextrin; cellulose derivatives such as crystalline cellulose; gum arabic; dextran; or pullulan; or inorganic excipients including silicate derivatives such as light anhydrous silicic acid, synthetic aluminium silicate, calcium silicate or magnesium aluminometasilicate; phosphates such as calcium hydrogenphosphate; carbonates such as calcium carbonate; or sulfates such as calcium sulfate can be listed), lubricants (for example, stearic acid or metal salts of stearic acid such as calcium stearate or magnesium stearate; talc; waxes such as beeswax or spermaceti wax; boric acid; adipic acid; sulfates such as sodium sulfate; glycol; fumaric acid; sodium benzoate; DL-leucine; laurylsulfates such as sodium lauryl sulfate or magnesium lauryl sulfate; silicates such as silicic anhydride or silicic hydrate; or the starch derivatives described above can be listed), binders (for example, hydroxypropylcellulose, hydroxypropylmethylcellulose, polyvinylpyrrolidone, macrogol can be listed), disintegrants (for example, cellulose derivatives such as low-substituted hydroxypropylcellulose, carboxymethylcellulose, calcium carboxymethylcellulose or internally crosslinked sodium carboxymethylcellulose; or chemically modified starch/cellulose derivatives such as carboxymethylstarch, sodium carboxymethylstarch or bridged polyvinylpyrrolidone; or the starch derivatives described above can be listed), emulsifiers (for example, colloidal clays such as bentonite or veegum; metal hydroxides such as magnesium hydroxide or aluminium hydroxide; anionic surfactants such as sodium lauryl sulfate or calcium stearate; cationic surfactants such as benzalkonium chloride; or nonionic surfactants such as polyoxyethylene alkyl ether, polyoxyethylene sorbitan fatty acid ester or sucrose esters of fatty acids can be listed), stabilizers (for example, paraoxybenzoate esters such as methyl paraben or propyl paraben; alcohols such as chlorobutanol, benzyl alcohol or phenylethyl alcohol; benzalkonium chloride; phenols such as phenol or cresol; thimerosal; dehydroacetic acid; or sorbic acid can be listed), flavour and odour correctives (for example, commonly used sweeteners, sour flavourings or fragrances can be listed) or diluents can be listed, but are not restricted to these additives.

Although the dosage of the medicament of the present invention can be selected in a suitable manner depending on various factors such as administration routes, types of active ingredients, patients' age, body weight or symptom, purposes of the prevention or treatment, an adult daily dosage that can be administered as the weight of the angiotensin II receptor antagonist is in general within the range of 0.001 to 1,000 mg, and preferably 0.1 to 500 mg.

### [Examples]

The present invention will be hereinafter described in more detail by the Examples and Preparation examples, but the scope of the present invention should not be limited to the following examples.

### Example 1

Seven-day-old C57BL/6 mice were housed for five days FP0410epdivxl with their mother in roughly 75% oxygen. Subsequently, the animals were housed for another five days after returning to ordinary air. While housing in air, the animals were administered a test compound once a day. Following completion of administration, the animals were sacrificed by dislocation of the cervical vertebra followed by excision of the eyeball. The eyeballs were fixed in neutral formalin. After preparing flat specimens of the retina, vascular endothelium was stained by ADPase staining (Lutty, et al., Arch. Ophthalmol. 1992; 100: 267). After staining, retinopathy was scored in a blind study, and retinopathy was assessed for each of 12 retinal sections obtained by dividing the entire circumference of the retina into 12 equal sections centering about the papilla of the optic nerve. Assessments were made by scoring either the presence of retinopathy (score: 1) or the absence of retinopathy (score: 0), and determining the retinopathy score for each specimen based on the total score of the 12 sections. The score of each test compound was calculated as a percentage based on assigning a value of 100% for the score following administration of solvent. The test compounds consisted of (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate (hereinafter, indicated as "Compound A") for the medicament of the present invention, Candesartan cilexetil and Losartan.

**Table 1**

| Test Compound | Dosage (mg/kg) | Retinopathy (%) | p Value |
|---|---|---|---|
| Control (solvent) | | 100 | |
| Compound A | 1 | 57 ± 13 | p<0.01 |
| | 3 | 40 ± 9 | p<0.001 |
| | 10 | 31 ± 7 | p<0.001 |
| | 30 | 32 ± 11 | p<0.001 |
| Candesartan cilexetil | 1 | 84 ± 14 | n.s. |
| | 3 | 70 ± 12 | n.s. |
| | 10 | 56 ± 11 | p<0.05 |
| | 30 | 58 ± 11 | p<0.05 |
| Losartan | 3 | 90 ± 11 | n.s. |
| | 10 | 54 ± 10 | p<0.01 |
| | 30 | 42 ± 9 | p<0.001 |
| | 100 | 40 ± 11 | p<0.001 |

| | | | |
|---|---|---|---|
| n = 9 to 16 n.s. indicates "not significant", and refers to the absence of statistical significance. | | | |

### Example 2

Male New Zealand White rabbits were used in this example. The animals were anesthetized by administering suitable amounts of animal Ketaral (registered trade mark) 50 and Celactal (registered trade mark). Moreover, the eyes were topically anesthetized by dropping 0.4 % Benoxil (registered trade mark) ophthalmic solution. The procedure was basically performed in accordance with the method of Ziche, et al. (J. Clin. Invest. 1997 99: 2625-2634), and consisted of creating a pocket in the centre of the cornea with a razor, and inserting a hydron pellet containing VEGF produced in advance into the pocket. A solution of the test compound was administered once a day for one week. Corneal neovascularization was photographed on the day after final administration of the test compound. Neovascularization was evaluated using the density and length of newly formed vessels as an index. Namely, neovascularization density was scored from 0 to 5 by referring to standard photographs corresponding to each score. The length of neovascularization was calculated by measuring the length (mm) of vessels uniformly extending from the corneal limbus towards the pellet based on a scale bar on the photograph. The value obtained by multiplying the neovascularization density score by the neovascularization length (mm) was used to indicate angiogenic activity (AA).

**Table 2**

| Test Compound | Dosage (mg/kg) | Angiogenic Activity (AA) | p Value |
|---|---|---|---|
| Control (solvent) | | 0.72 ± 0.15 | |
| Compound A | 10 | 0.14 ± 0.11 | p<0.05 |

| | | | |
|---|---|---|---|
| n = 3 | | | |

**Preparation Example 1 - Capsule**

| | |
|---|---|
| Compound A | 20.0 mg |
| Lactose | 158.7 mg |
| Corn starch | 70.0 mg |
| Magnesium stearate | 1.3 mg |
| Total | 250 mg |

Powders of the above formula were mixed and passed through a 60 mesh sieve followed by filling the powder into a 250 mg No. 3 capsule to prepare the capsule.

**Preparation Example 2 - Tablet**

| | |
|---|---|
| Compound A | 20. 0 mg |
| Lactose | 154.0 mg |
| Corn starch | 25.0 mg |
| Magnesium stearate | 1.0 mg |
| Total | 200 mg |

Powders of the above formula were mixed and tableted with a tableting machine to prepare the tablet containing 200 mg per tablet. The tablet can be sugar-coated, if necessary.

### [Industrial applicability]

The medicament of the present invention comprising an angiotensin II receptor antagonist as its active ingredient is useful for the prevention or treatment of an intraocular angiogenic disease that is proliferative retinopathy, retinal vein occlusion, retinal artery occlusion or age-related macular degeneration, by oral administration.

## Claims

1. A medicament comprising (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate as its active ingredient, for use in the prevention or treatment of an intraocular angiogenic disease that is proliferative retinopathy, retinal vein occlusion, retinal artery occlusion or age-related macular degeneration, by oral administration.

2. The use of (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate, in the manufacture of a medicament for the prevention or treatment of an intraocular angiogenic disease that is proliferative retinopathy, retinal vein occlusion, retinal artery occlusion or age-related macular degeneration, by oral administration.

## Patentansprüche

1. Medikament, umfassend (5-Methyl-2-oxo-1,3-dioxolen-4-yl)methyl-4-(1-hydroxy-1-methylethyl)-2-propyl-1-[2'-(1H-tetrazol-5-yl)biphenyl-4-ylmethyl]imidazol-5-carboxylat als seinen Wirkstoff, zur Verwendung bei der Vorbeugung oder Behandlung einer intraokularen angiogenen Erkrankung, welche proliferative Retinopathie, retinaler Venenverschluss, retinaler Arterienverschluss oder altersbedingte Makuladegeneration ist, durch orale Verabreichung.

2. Verwendung von (5-Methyl-2-oxo-1,3-dioxolen-4-yl)methyl-4-(1-hydroxy-1-methylethyl)-2-propyl-1-[2'-(1H-tetrazol-5-yl)biphenyl-4-ylmethyl]imidazol-5-carboxylat, bei der Herstellung eines Medikaments zur Vorbeugung oder Behandlung einer intraokularen angiogenen Erkrankung, welche proliferative Retinopathie, retinaler Venenverschluss, retinaler Arterienverschluss oder altersbedingte Makuladegeneration ist, durch orale Verabreichung.

## Revendications

1. Médicament comprenant le 4-(1-hydroxy-1-méthyléthyl)-2-propyl-1-[2'-(1H-tétrazol-5-yl)biphényl-4-ylméthyl]imidazole-5-carboxylate de (5-méthyl-2-oxo-1,3-dioxolén-4-yl)méthyle comme son ingrédient actif, pour une utilisation dans la prévention ou le traitement d'une maladie angiogène intraoculaire qui est une rétinopathie proliférative, une occlusion veineuse rétinienne, une occlusion artérielle rétinienne ou une dégénérescence maculaire liée à l'âge, par une administration orale.

2. Utilisation du 4-(1-hydroxy-1-méthyléthyl)-2-propyl-1-[2'-(1H-tétrazol-5-yl)biphényl-4-ylméthyl]imidazole-5-carboxylate de (5-méthyl-2-oxo-1,3-dioxolén-4-yl)méthyle, dans la fabrication d'un médicament pour la prévention ou le traitement d'une maladie angiogène intraoculaire qui est une rétinopathie proliférative, une occlusion veineuse rétinienne, une occlusion artérielle rétinienne ou une dégénérescence maculaire liée à l'âge, par une administration orale.
